# EUROPÄISCHE PATENTSCHRIFT

(11) **EP 1 322 282 B1**
(45) Veröffentlichungstag und Bekanntmachung des Hinweises auf die Patenterteilung: **17.11.2004**
(21) Anmeldenummer: 01985671.5
(22) Anmeldetag: 18.09.2001
(51) Int. Cl.: A61K 7/16, A61K 7/22

(54) **ZAHNREINIGUNGSMITTEL**
DENTAL CLEANING AGENTS
PRODUITS DE NETTOYAGE POUR LES DENTS

(30) Priorität: 27.09.2000 DE 10047760
(43) Veröffentlichungstag der Anmeldung: 02.07.2003
(73) Patentinhaber: Henkel Kommanditgesellschaft auf Aktien, 40589 Düsseldorf (DE)
(72) Erfinder: EISFELD, Wolf, D-40597 Düsseldorf (DE); BRANDS, Angela, D-45481 Mühlheim/Ruhr (DE); PASTURA, Amerigo, D-58453 Witten (DE); WÜLKNITZ, Peter, D-42799 Leichlingen (DE)
(86) Internationale Anmeldenummer: PCT/EP2001/010766
(87) Internationale Veröffentlichungsnummer: WO 2002/026203

(56) Entgegenhaltungen:
- EP-A- 0 692 246
- WO-A-99/09942
- WO-A-99/63959
- DE-A- 19 911 055
- US-A- 5 911 981

## Beschreibung

Die Erfindung betrifft Zahnreinigungsmittel in Form einer wäßrigen, pastösen oder flüssigen Dispersion, mit einem Gehalt von teilchenförmigen Poliermittelkomponenten, Feuchthaltemitteln und Tensiden, die als Tenside eine Kombination von anionischen Tensiden, Betaintensiden und Polyoxyethylen-Sorbitanestem enthalten sowie die Verwendung dieser Zahnreinigungsmittel zur Verbesserung der Schaumkonsistenz.

Zahnpasten, Zahncremes und Zahngele werden bei der täglichen Reinigung der Zähne durch Bürsten mit einer Zahnbürste angewendet. Die Zahnreinigungsmittel sollen in erster Linie die Reinigung der Zahnoberfläche von Speiseresten, Verfärbungen durch z. B. Tabak oder Tee und von den fest anhaftenden bakteriellen Zahnbelägen, der sogenannten Plaque, unterstützen. Dies geschieht einerseits durch die mechanische Wirkung der Bürste und der Poliermittelkomponenten, andererseits durch die grenzflächenaktive und dispergierende Wirkung der Tenside. Darüber hinaus sollen die Tenside einen gewissen Schaum erzeugen, der während des Zähneputzens die Komponenten der Zahnpaste gleichmäßig im Mund verteilt und beim Verwender auch ein durchaus angenehmes Mundgefühl hervorruft. So ist die Konsistenz des beim Zähneputzen gebildeten Schaums ein wichtiger Faktor für die Produktakzeptanz. Eine zu schnelle Verflüssigung des Schaums läßt diesen vorzeitig aus dem Mund tropfen, was als unangenehm empfunden wird und dazu führt, daß das Zähneputzen frühzeitig abgebrochen wird. Demgegenüber wäre es aber zahnmedizinisch sinnvoll, das Zähneputzen über einen Zeitraum von wenigstens drei Minuten auszudehnen.

Die Problematik des zu geringen und zu wenig konsistenten Schaums beim Zähneputzen zeigt sich insbesondere bei halbflüssigen Zahnreinigungsmitteln, die oft auch transparent oder klar formuliert sind, und die einen hohen Anteil an Feuchthaltemitteln enthalten.

Die naheliegendste Lösung des Problems bestünde in einer Erhöhung der Tensidkonzentration. Es hat sich aber gezeigt, daß diese Maßnahme nur zu einer mäßigen Verbesserung der Schaumkraft führt. Andererseits sind einer Erhöhung der Konzentration z. B. der schaumstarken anionischen Tenside schon aus dermatologischen und geschmacklichen Gründen enge Grenzen gesetzt.

Aus DE 19845274 sind flüssige, klare Zahnreinigungsgele bekannt, die als Tensidkomponenten eine Kombination aus Natriumlaurylsulfat, Betaintensid und nichtionischen Tensiden enthalten. Auch in W097146124 A1 sind Zahnpflegemittel beschrieben, die anionische Tenside und nichtionische Tenside in einem bestimmten Gewichtsverhältnis zueinander enthalten. Auch WO98/55084 A1 beschreibt Zahnreinigungsmittel, die neben anionischen Tensiden hohe Mengen an nichtionogenen Tensiden zur Verbesserung der Reinigungsleistung enthalten.

Die Zahnpasten des zitierten Standes der Technik sind aber in ihrer Schaumentwicklung noch nicht befriedigend. Es wurde nun gefunden, daß das Schäumvermögen solcher Zahnreinigungsmittel erheblich gesteigert werden kann, wenn als Tensidkomponente eine Kombination von anionischen, betainischen und nichtionischen Tensiden in einem speziellen Gewichtsverhältnis, mit einem hohen Anteil an ethoxylierten Fettsäurepolyolmonoestern enthalten ist.

Gegenstand der Erfindung sind wäßrige Zahnreinigungsmittel mit einem Gehalt an teilchenförmigen Poliermittelkomponenten, Feuchthaltemitteln und Tensiden, die als Tenside eine Kombination von anionischen Tensiden (A), Betaintensiden (B) und ethoxylierten Fettsäure-Polyolmonoestern (C) enthalten, dadurch gekennzeichnet, daß die Tensidkomponenten im Verhältnis (A):(B):(C)=1:(0,1-1):(0,5-2) und in einer Menge von (A+B+C)=2-5 Gew.%, bezogen auf das Gesamtgewicht des Zahnreinigungsmittels, enthalten sind.

Als Poliermittelkomponenten können alle hierfür bekannten, teilchenförmigen Poliermittel, bevorzugt aber Fällungs- und Gelkieselsäuren, Aluminiumhydroxid, Aluminiumoxid, Calciumpyrophosphat, Calciumhydrogenphosphat, Kreide, Hydroxylapatit, Natriumaluminiumsilikat (z. B. Zeolith A) oder organische Polymere z. B. Polymethacrylate, eingesetzt werden.

In einer bevorzugten Ausführung der Erfindung sind als Poliermittelkomponenten Kieselsäure-Poliermittel enthalten. Als Kieselsäure-Polierkomponenten eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20 C, gemessen mit: Brookfiled-Viskosimeter RVF, Helipath, Spindel, TE 4UpM, nach 1 Stunde) in einer Menge von 10 - 20 Gew.-% des Zahngels. Bevorzugt geeignete Fällungskieselsäuren dieser Art weisen außerdem gerundete Ecken und Kanten auf und sind unter der Handelsbezeichnung Sident®12 DS (DEGUSSA) erhältlich. Eine weitere geeignete Kieselsäure ist Zeodent 113 (Huber Corp) mit einer BET-Oberfläche von 150-250 m²/g.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahngele gemäß der vorliegenden Erfindung.

Als Feuchthaltemittel können Glycerin, Sorbit, Xylit, Propylenglykol, Polyethylenglycol oder Mischungen dieser Stoffe eingesetzt werden. Als Polyethylenglykole sind bevorzugt solche mit mittleren Molekulargewichten von 200 bis 2000 geeignet.

Als anionische Tenside (A) sind bevorzugt die gut wasserlöslichen, stark schäumenden primären linearen Alkyl (C₁₂-C₁₈)-sulfate, die linearen C₁₂-C₁₈-Alkansulfonate oder Alpha-Olefinsulfonate, Sulfobernsteinsäuremono (C₁₂-C₁₈)-alkylester-Salze, Sulfoessigsäure (C₁₂-C₁₈)-alkylester-Salze, 2-Hydroxy-ethansulfonsäure (C₁₂-C₁₈)-alkylester (Acylisethionate), Fettsäure-(C₁₂-C₁₈)-monoglycerid-sulfate, C₁₂-C₁₈-Acylsarkoside- oder Acyltauride, jeweils in Form ihrer wasserlöslichen Alkalisalze, insbesondere der Natriumsalze, enthalten. Eine bevorzugte Rolle spielen dabei die linearen C₁₂-C₁₈-Alkylsulfate, insbesondere das Natrium-Laurylsulfat.

Als Betaintenside (B) sind alle leicht wasserlöslichen Tenside geeignet, die eine zwitterionische Struktur mit dem Element 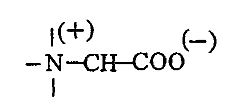 aufweisen. Geeignete Betaintenside sind insbesondere N-(C₁₂-C₁₈)-Alkyl-dimethyl-acetobetain und N-(C₁₂-C₁₈)-Acylaminopropyl-acetobetain (Cocoamidopropyl-Betaine).

Als ethoxylierte Fettsäure-Polyolmonoester eignen sich alle Anlagerungsprodukte von 5 - 30 Mol Ethylenoxid an Monoester linearer C₁₂-C₁₈-Fettsäuren und Glycerin, Diglycerin oder Anhydrosorbit. Geeignete ethoxylierte Fettsäure-Glycerinmonoester sind unter der Handelsbezeichnung Tagat® (Tego Cosmetics), geeignete ethoxylierte Fettsäure-Sorbitanmonoester sind unter der Handelsbezeichnung Tween® (ICI Surfactants) erhältlich.

Als besonders effektiv zur Verbesserung des Schäumvermögens eignet sich ein Polyoxyethylen-Sorbitanmonooleat, insbesondere das Anlagerungsprodukt von 20 Mol Ethylenoxid an Sorbitanmonooleat (Tween® 80, Polysorbate 80 Lamesorb® SMO 20). In einer besonders bevorzugten Ausführung ist als ethoxylierter Fettsäure-Polyolmonoester ein Polyoxyethylen-Sorbitanmonooleat enthalten.

Der Gehalt an ethoxylierten Fettsäure-Polyolmonoestern beträgt bevorzugt wenigstens 0,5 Gew.-% der erfindungsgemäßen Zahnreinigungsmittel, ganz besonders bevorzugt ist ein Gehalt von 0,5 - 2 Gew.-%, jeweils bezogen auf das Gesamtgewicht des Zahnreinigungsmittels, an ethoxylierten Fettsäure-Polyolenmonoestern.

Wie weiter oben bereits ausgeführt, können die erfindungsgemäßen Zahnpflegemittel auch als flüssige oder halbflüssige Dispersionen mit Viskositäten (bei 20 °C) von 5 - 100 Pa·s formuliert werden. Zu diesem Zweck ist es erforderlich, den Gehalt an Polierkomponenten zu begrenzen und auf Verdickungskieselsäuren zu verzichten.

In einer bevorzugten Ausführungsform enthält das erfindungsgemäße wäßrige Zahnreinigungsmittel als Polierkomponente Kieselsäuren.

Bevorzugt wird die Kieselsäurekomponente in einer Menge von 8 - 20 Gew.%, insbesondere in einer Menge von 10 - 15 Gew.%, bezogen auf das Gesamtgewicht des Zahnreinigungsmittels, eingesetzt.

Weiterhin wird bevorzugt in den erfindungsgemäßen Mitteln als Feuchthaltemittel eine Komponente, die ausgewählt ist aus Sorbit, Glycerin, Propylenglycol, Polyethylenglycol und Gemischen davon, eingesetzt.

Besonders bevorzugt wird das Feuchthaltemittel in einer Menge von 40 - 70 Gew.%, bezogen auf das Gesamtgewicht des Zahnreinigungsmittels, eingesetzt.

Die erfindungsgemäßen wäßrigen Zahnpflegemittel können auch als transparente oder klar durchsichtige Gele formuliert werden. Zu diesem Zweck ist es erforderlich, daß das Trägergemisch aus Wasser und Feuchthaltemitteln einen Brechungsindex aufweist, der sich von dem Brechungsindex des Poliermittels möglichst wenig unterscheidet. Bei Verwendung von Kieselsäure-Poliermitteln kann dies in der Regel durch Feuchthaltemittelgehalte von 40 - 60 Gew.-% und ein Gewichtsverhältnis von Feuchthaltemittel zu Wasser von (1,5 - 2,5) : 1 erreicht werden. Dabei ist es bevorzugt, daß wenigstens die Hälfte des Feuchthaltemittels aus Sorbit besteht und ein Polyethylenglycol mit einem Molgewicht von 200 - 2000 in einer Menge von 1 - 5 Gew.-% enthalten ist.

Zusätzlich zu den genannten obligatorischen Komponenten können die erfindungsgemäßen Zahnpflegemittel weitere übliche Inhaltsstoffe sowie Hilfs- und Zusatzstoffe enthalten, die den therapeutischen oder mundhygienischen Wert erhöhen oder die organoleptischen und anwendungstechnischen Eigenschaften verbessern.

Solche weiteren Inhaltsstoffe sind z. B. Wirkstoffe gegen Zahn- und Zahnfleischerkrankungen, beispielsweise Fluorverbindungen wie Na-Fluorid, Zink-fluorid und Na-Monofluorphosphat, Antizahnsteinwirkstoffe, (z. B. Polyphosphate, Hydroxyethan-diphosphonate, Azacycloheptandiphosphonat), antimikrobielle Stoffe (z. B. Biguanide, Chlorhexidin, Triclosan), Vitamine (z. B. Tocopherol, Retinol), Panthenol, Pflanzen- und Algenextrakte sowie wundheilende und entzündungshemmende Stoffe, wie z. B. Harnstoff, Allantoin, Azulen, Rhodanide,Magnesiumsalze.

Als Hilfs- und Zusatzstoffe können z. B. enthalten sein
- Bindemittel, wie z. B. natürliche und/oder synthetische wasserlösliche Polymere wie Alginate, Carragheenate, Traganth, Stärke und Stärkeether, Celluloseether, wie z. B. Carboxymethylcellulose. Hydroxyethylcellulose, Methylhydroxypropylcellulose, Guar, Akaziengum, Agar-Agar, Xanthan-Gum, Succinoglucan-Gum, Johannisbrotmehl, Pektine, wasserlösliche Carboxyvinylpolymere (Carbopol®), Polyvinylalkohol, Polyvinylpyrrolidon und Polyethylenglycole mit Molekulargewichten von 2000 - 5000000.
- Geschmacksstoffe, z. B. Aromen wie Pfefferminzöl, Krauseminzöl, Eukalyptusöl, Anisöl, Fenchelöl, Kümmelöl, Menthylacetat, Zimtaldehyd, Anethol, Vanillin, Thymol und andere natürliche oder naturidentische Öle oder synthetische Geschmacksstoffe.
- Süßungsmittel, wie z. B. Saccharin-Natrium, Natrium-Cyclamat, Acesulfam, Aspartam, Sterioxide, Thaumatin, Glycyrrhicin, Sucrose, Lactose, Maltose oder Fructose.
- Konservierungsmittel, wie z. B. p-Hydroxybenzoesäureester, Natriumbenzoat, Natriumsorbat
- Puffersubstanzen, wie z. B. primäre, sekundäre und tertiäre Akaliphosphate, Citronensäure-/Natriumcitrat
- Remineralisierende Salze, wie z. B. Calciumdihydrogenphosphat, Magnesium-, Zink oder Mangansalze
- Farbstoffe und/oder Pigmente
Ein weiterer Gegenstand der Erfindung ist die Verwendung der erfindungsgemäßen wäßrigen Zahnreinigungsmittel zur Verbesserung der Schaumkonsistenz.

Die folgenden Beispiele sollen den Erfindungsgegenstand näher erläutern:

### Beispiele

### Prüfung der Schaumeigenschaften von Zahncremes

### 1. Prüfmethodik

Zur vergleichenden Prüfung der Schaumeigenschaften von Zahnpflegemitteln wurde ein Zahnputz-Simulator verwendet, der wie folgt aufgebaut ist:

An einem pneumatisch betriebenen Linearvorschub ist eine Zahnbürste befestigt, die mit variablem Druck auf einem Wellenprofil aus Stahlzylindern positioniert wird. Die Verfahrstrecke pro Hub beträgt 11cm. Das Wellenprofil wurde anhand unterschiedlicher Anforderungen konzipiert (Abriebfestigkeit, regelmäßige Abstände, keine steilen Kanten, leichte Reinigung nach den Versuchen) und lehnt sich an das natürliche Profil der Zähne im Mund an. Darüber hinaus ist es abnehmbar, um die Reinigung zu erleichtern. Die Höhenverstellung der Zahnbürste, die den Andruck der Bürste auf dem Wellenprofil regelt, läßt sich arretieren. Innerhalb einer Putzzeit von 60s werden 2ml Wasser über eine Schlauchpumpe kontinuierlich zudosiert. Das Wasser wird dabei direkt neben dem Bürstenkopf zugetropft. Die Apparatur läßt sich mit einem Schaltkasten hinsichtlich der Anzahl der Bewegungen oder über eine definierte Zeitdauer steuern. Zusätzlich kann am Pneumatikzylinder die Geschwindigkeit eingestellt werden. Dies bietet unterschiedliche Möglichkeiten der Versuchsgestaltung. Bei den beschriebenen Versuchsreihen entsprechen beispielsweise 16 Hübe einer Zeitdauer von 10s.

Die Prüfungen wurden wie folgt durchgeführt:

Zunächst wird 1g Produkt über eine Strecke von 3,5 cm auf das Wellenprofil dosiert, sodann die Profilplatte in die Maschine eingesetzt, die angefeuchtete Zahnbürste mit leichtem Druck aufgesetzt und das erste Bild ohne Schaum als Referenz aufgenommen (O-Bild). Nach dem Start der Maschine werden 16 Hin- und Her-Bewegungen der Zahnbürste (d. h. 8x hin und 8x zurück; Dauer insgesamt 10s) ausgeführt. Nach dem Stoppen der Bewegung wird das nächste Bild aufgenommen, usw.. Dieser Vorgang wird noch fünfmal wiederholt, bis insgesamt 60s Putzzeit erreicht sind. In der gesamten Putzperiode wird, wie bereits beschrieben, kontinuierlich eine Gesamtmenge Wasser von 2 ml zudosiert. Nach Abschluß dieses Schaumerzeugungs-Vorganges wird 5 min lang jede Minute ein Bild aufgenommen, um die Schaumstabilität zu dokumentieren.

### 2. Auswertung:

Auf allen Bildern wird mit Algorithmen der digitalen Bildverarbeitung der durch den Schaum erzeugt helle Flächenanteil detektiert und prozentual ins Verhältnis zur gesamten Meßfläche gesetzt. Der jeweils beim O-Bild ermittelte Flächenanteil wird von allen folgenden Meßwerten abgezogen, da a) bei diesem noch kein Schaum erzeugt wurde und helle Areale somit nur Artefakte sind und b) um eine Normierung auf einheitliche Anfangswerte zu erreichen.

In der Tabelle I sind als Schaum (60 sec) und Schaum (5 min) die prozentualen Flächenanteile des Schaumes nach 60 Sekunden und nach 5 Minuten angegeben.

### 3. Durchführung

Es wurden fünf Zahnpasten, die sich nur in der Zusammensetzung der Tensidkomponenten unterschieden, einer vergleichenden Prüfung unterzogen.

Die Vergleichspaste Va enthält weniger als das beanspruchte Mengenverhältnis an Polyoxyethylenglycerylmonostearat.

Die Vergleichspaste Vb enthält eine zusätzliche Menge (1 Gew.-%) an Na-Laurylsulfat. Die Vergleichspaste Vc enthält eine zusätzliche Menge (1 Gew.-% Aktivsubstanz) an Betaintensid.

Die erfindungsgemäßen Pasten 1 und 2 weisen eine deutlich höhere Schäumkraft und Schaumstabilität auf. Die Zusammensetzung der Zahnpasten und die Ergebnisse der Schaummessungen sind der Tabelle zu entnehmen. Die darin enthaltenen Mengenangaben beziehen sich, sofern nicht anders angegeben, auf die Einsatzmenge in g).

| | **V a** | **V b** | **V c** | **1** | **2** |
|---|---|---|---|---|---|
| Sident® 8 | 12,0 | 12,0 | 12,0 | 12,0 | 12,0 |
| Sorbit 70%ig | 30,0 | 30,0 | 30,0 | 30,0 | 30,0 |
| Glycerin 86%ig | 33,0 | 33,0 | 33,0 | 33,0 | 33,0 |
| Lipoxol® 1550 | 1,0 | 1,0 | 1,0 | 1,0 | 1,0 |
| Na-Saccharinat | 0,2 | 0,2 | 0,2 | 0,2 | 0,2 |
| Na-Benzoat | 0,5 | 0,5 | 0,5 | 0;5 | 0,5 |
| Na₂ HPO₄ | 0,5 | 0,5 | 0,5 | 0,5 | 0,5 |
| Na-Fluorid | 0,32 | 0,32 | 0,32 | 0,32 | 0,32 |
| Mg SO₄ · 7 H₂O | 0,21 | 0,21 | 0,21 | 0,21 | 0,21 |
| Zn SO₄ · 7 H₂O | 0,1 | 0,1 | 0,1 | 0,1 | 0,1 |
| Mn SO₄ · 7 H₂O | 0,01 | 0,01 | 0,01 | 0,01 | 0,01 |
| Triclosan | 0,2 | 0,2 | 0,2 | 0,2 | 0,2 |
| Xanthan-Gum | 0,55 | 0,55 | 0,55 | 0,55 | 0,55 |
| Aroma | 1,0 | 1,0 | 1,0 | 1,0 | 1,0 |
| Texapon® K 1296 | 1,5 | 2,5 | 1,5 | 1,5 | 1,5 |
| Tego® Betain BL 215 | 0,6 | 0,6 | 3,8 | 0,6 | 0,6 |
| Tagat®S | 0,5 | 0,5 | 0,5 | 1,5 | 0,5 |
| Lamesorb® SMO 20 | ----- | ----- | ----- | ----- | 1,0 |
| Ethanol | 2,0 | 2,0 | 2,0 | 2,0 | 2,0 |
| Wasser | ad 100 | ad 100 | Ad 100 | ad 100 | ad 100 |
| % Schaumverbesserung | | | | | |
| nach 60 sec | ± 0 | + 5,9 | + 2,3 | + 10,9 | + 11,7 |
| nach 5 min Ruhe | ± 0 | + 12,0 | + 12,2 | + 18,3 | + 19,1 |

Es wurden folgende Handelsprodukte verwendet:
- Lipoxol®1550:: Polyethylenglycol, Molgew. 1550
- Texapon® K1296:: Natrium-Laurylsulfat
- Tego® Betain BL 215:: Cocoamidopropyl Betaine (30%ig)
- Tagat® S:: PEG-30-Glyceryl Stearate
- Lamesorb® SMO 20:: PEG-20-Sorbitan-Monolaurate

## Patentansprüche

1. Wäßrige Zahnreinigungsmittel mit einem Gehalt an teilchenförmigen Polierkomponenten, Feuchthaltemitteln und Tensiden, die als Tenside eine Kombination von anionischen Tensiden (A), Betaintensiden (B) und ethoxylierten Fettsäure-Polyolmonoestem (C) enthalten, **dadurch gekennzeichnet, daß** die Tensidkomponenten im Gewichtsverhältnis (A) : (B) : (C) = 1 : (0,1 - 1) : (0,5 - 2) und in einer Menge von (A+B+C) = 2 - 5 Gew.-%, bezogen auf das Gesamtgewicht des Zahnreinigungsmittels, enthalten sind.

2. Wäßrige Zahnreinigungsmittel gemäß Anspruch 1, **dadurch gekennzeichnet, daß** als ethoxylierter Fettsäure-Polyolmonoester ein Polyoxyethylen-Sorbitanmonooleat enthalten ist.

3. Wäßrige Zahnreinigungsmittel gemäß einem der Ansprüche 1 oder 2, **dadurch gekennzeichnet, daß** der Gehalt an ethoxylierten Fettsäure-Polyolmonoestem wenigstens 0,5 Gew.-% beträgt.

4. Wäßriges Zahnreinigungsmittel gemäß einem der Ansprüche 1 bis 3, **dadurch gekennzeichnet, daß** mindestens eine Poliermittelkomponente aus der Gruppe der Kieselsäuren ausgewählt ist.

5. Wäßriges Zahnreinigungsmittel gemäß Anspruch 4, **dadurch gekennzeichnet, daß** die Poliermittelkomponente in einer Menge von 10 - 15 Gew.-%, bezogen auf das Gesamtgewicht des Zahnreinigungsmittels, enthalten ist.

6. Wäßriges Zahnreinigungsmittel gemäß einem der Ansprüche 1 bis 3, **dadurch gekennzeichnet, daß** das Feuchthaltemittel ausgewählt ist aus Sorbit, Glycerin, Polyethylenglycol und Gemischen davon.

7. Wäßriges Zahnreinigungsmittel gemäß Anspruch 6, **dadurch gekennzeichnet daß** das Feuchthaltemittel in einer Menge von 40 - 70 Gew.-%, bezogen auf das Gesamtgewicht des Zahnreinigungsmittels, enthalten ist.

8. Verwendung eines wäßrigen Zahnreinigungsmittels gemäß einem der Ansprüche 1 bis 7 zur Verbesserung der Schaumkonsistenz.

## Claims

1. A water-based tooth cleaning preparation containing particulate polishing components, humectants and surfactants, the surfactants being a combination of anionic surfactants (A), betaine surfactants (B) and ethoxylated fatty acid polyol monoesters (C), **characterized in that** the surfactant components are present in a ratio by weight of (A) to (B) to (C) of 1:(0.1-1):(0.5-2) and in a quantity of (A+B+C) of 2 to 5% by weight, based on the total weight of the tooth cleaning preparation.

2. A water-based tooth cleaning preparation as claimed in claim 1, **characterized in that** a polyoxyethylene sorbitan mono-oleate is present as the ethoxylated fatty acid polyol monoester.

3. A water-based tooth cleaning preparation as claimed in claim 1 or 2, **characterized in that** the content of ethoxylated fatty acid polyol monoesters is at least 0.5% by weight.

4. A water-based tooth cleaning preparation as claimed in any of claims 1 to 3, **characterized in that** at least one polishing component is selected from the group of silicas.

5. A water-based tooth cleaning preparation as claimed in claim 4, **characterized in that** the polishing component is present in a quantity of 10 to 15% by weight, based on the total weight of the tooth cleaning preparation.

6. A water-based tooth cleaning preparation as claimed in any of claims 1 to 3, **characterized in that** the humectant is selected from sorbitol, glycerol, polyethylene glycol and mixtures thereof.

7. A water-based tooth cleaning preparation as claimed in claim 6, **characterized in that** the humectant is present in a quantity of 40 to 70% by weight, based on the total weight of the tooth cleaning preparation.

8. The use of the water-based tooth cleaning preparation claimed in any of claims 1 to 7 for improving foam consistency.

## Revendications

1. Produits aqueux de nettoyage pour les dents présentant une teneur en composants de polissage en forme de particules, des agents de rétention d'humidité et des agents tensioactifs qui contiennent comme agents tensioactifs une combinaison d'agents tensioactifs anioniques (A), d'agents tensioactifs de bétaïne (B) et de monoesters éthoxylés d'acide gras et de polyol (C), **caractérisés en ce que** les composants tensioactifs sont contenus dans un rapport pondéral (A) : (B) : (C) = 1 : (0,1 - 1) : (0,5 - 2) et en une quantité de (A+B+C) = 2 à 5% en poids par rapport au poids total de l'agent de nettoyage pour les dents.

2. Produits aqueux de nettoyage pour les dents selon la revendication 1, **caractérisés en ce qu'**ils contiennent comme monoester éthoxylé d'acide gras et de polyol un monooléate de polyoxyéthylène et de sorbitol.

3. Produits aqueux de nettoyage pour les dents selon l'une quelconque des revendications 1 ou 2, **caractérisés en ce que** la teneur en monoesters éthoxylés d'acide gras et de polyol est d'au moins 0,5% en poids.

4. Produit aqueux de nettoyage pour les dents selon l'une quelconque des revendications 1 à 3, **caractérisé en ce qu'**au moins un des composants de polissage est choisi dans le groupe des silices.

5. Produit aqueux de nettoyage pour les dents selon la revendication 4, **caractérisé en ce que** le composant de polissage est contenu en une quantité de 10 à 15% en poids par rapport au poids total du produit de nettoyage pour les dents.

6. Produit aqueux de nettoyage pour les dents selon l'une quelconque des revendications 1 à 3, **caractérisé en ce que** l'agent de rétention d'humidité est choisi parmi le sorbitol, le glycérol, le polyéthylèneglycol et les mélanges de ceux-ci.

7. Produit aqueux de nettoyage pour les dents selon la revendication 6, **caractérisé en ce que** l'agent de rétention d'humidité est contenu en une quantité de 40 à 70% en poids par rapport au poids total du produit de nettoyage pour les dents.

8. Utilisation d'un produit aqueux de nettoyage pour les dents selon l'une quelconque des revendications 1 à 7 pour améliorer la consistance de la mousse.
